(19)

Europäisches
Patentamt
European
Patent Office
Office européen
des brevets

(11) **EP 2 704 733 B1**

(12) **EUROPEAN PATENT SPECIFICATION**

(45) Date of publication and mention
of the grant of the patent:
**20.06.2018 Bulletin 2018/25**

(21) Application number: **12727221.9**

(22) Date of filing: **03.05.2012**

(51) Int Cl.:
*A61K 35/747* (2015.01)    *A23L 33/135* (2016.01)
*C12R 1/25* (2006.01)    *C12R 1/225* (2006.01)

(86) International application number:
**PCT/BG2012/000014**

(87) International publication number:
**WO 2012/149615 (08.11.2012 Gazette 2012/45)**

(54) **POLYBACTERIAL PREPARATION and method for obtaining thereof**

POLYBAKTERIELLE Zusammensetzung und Methode zur Herstellung

PRÉPARATION POLYBACTÉRIENNE et méthode de préparation

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **04.05.2011 BG 11093211**

(43) Date of publication of application:
**12.03.2014 Bulletin 2014/11**

(73) Proprietor: **LB Bulgaricum PLC
1000 Sofia (BG)**

(72) Inventors:
• **DIMITROV, Zhechko
1505 Sofia (BG)**
• **MICHAYLOVA, Michaela
1111 Sofia (BG)**

(56) References cited:
WO-A1-2010/054439    WO-A2-2009/138091
US-A- 6 159 465    US-A1- 2003 039 723
US-A1- 2004 106 171    US-A1- 2008 019 954
US-A1- 2011 091 431

• **Hyun Jeong Ryu; Chang-Ho Chung; Kyu Hang
Kyung: "Evaluation of nisin as a preservative to
prevent over-acidification of kimchi.", Food
Science and Biotechnology, 1 January 1998
(1998-01-01), pages 205-208, XP055018976,
Retrieved from the Internet:
URL:http://next.sejong.ac.kr/~kyungkh/dawn
load/evaluation%20of%20nisin.pdf [retrieved on
2012-02-09]**
• **ROBERT MARIE-CLAUDE ET AL: "Peptides
derived from sodium caseinate Hydrolysates
produced by Lactobacillus helveticus NCC 2765",
JOURNAL OF AGRICULTURAL AND FOOD
CHEMISTRY, vol. 52, no. 23, 16 October 2004
(2004-10-16), pages 6923-6931, XP002376051,
AMERICAN CHEMICAL SOCIETY, US ISSN:
0021-8561, DOI: 10.1021/JF049510T**
• **YAMAMOTO NAOYUKI ED - KENJI SONOMOTO
AND ATSUSHI YOKOTA (EDS): "CHAPTER 15:
Antihypertensive Metabolites from Lactic Acid
Bacteria", 1 January 2011 (2011-01-01), LACTIC
ACID BACTERIA AND BIFIDOBACTERIA:
CURRENT PROGRESS IN ADVANCED
RESEARCH, CAISTER ACADEMIC PRESS,
NORFOLK, US, XP009172001, ISBN:
978-1-904455-82-0 pages 223-232, abstract; table
15.1**
• **USMAN ET AL: "Bile Tolerance, Taurocholate
Deconjugation, and Binding of Cholesterol by
Lactobacillus gasseri Strains", JOURNAL OF
DAIRY SCIENCE, vol. 82, no. 2, 1 February 1999
(1999-02-01), pages 243-248, XP027045846,
AMERICAN DAIRY SCIENCE ASSOCIATION, US
ISSN: 0022-0302 [retrieved on 1999-02-01]**

**(Cont. next page)**

EP 2 704 733 B1

- **TOSHIHIRO SASHIHARA ET AL: "Effect of growth conditions of Lactobacillus gasseri OLL2809 on the immunostimulatory activity for production of interleukin-12 (p70) by murine splenocytes", INTERNATIONAL JOURNAL OF FOOD MICROBIOLOGY, vol. 120, no. 3, 1 December 2007 (2007-12-01), pages 274-281, XP055013338, ISSN: 0168-1605, DOI: 10.1016/j.ijfoodmicro.2007.09.003**
- **WANG C Y ET AL: "Effect of Lactobacillus-fermented adlay-based milk on lipid metabolism of hamsters fed cholesterol-enriched diet", FOOD RESEARCH INTERNATIONAL, vol. 43, no. 3, 1 April 2010 (2010-04-01), pages 819-824, XP026905639, ELSEVIER APPLIED SCIENCE, BARKING, GB ISSN: 0963-9969, DOI: 10.1016/J.FOODRES.2009.11.020 [retrieved on 2009-12-04]**
- **DATABASE WPI Week 200624 Thomson Scientific, London, GB; AN 2006-226321 XP002711651, -& JP 2006 075083 A (TOYO SHINYAKU KK) 23 March 2006 (2006-03-23)**

Remarks:

The file contains technical information submitted after the application was filed and not included in this specification

**Description**

**TECHNICAL FIELD**

This invention relates to the area of biotechnology and in particular to probiotic strains of lactic acid bacteria and the polybacterial preparation, which is obtained by them. This disclosure also relates to the complex beneficial health effects of the preparation and its use as a dietary supplement for prevention of various diseases.

**BACKGROUND ART**

Probiotics are live edible microorganisms that have a positive effect on human health. Most probiotics are lactobacilli and / or bifidobacteria. They are non-pathogenic bacteria able to colonize on the gastrointestinal tract. It has been shown that lactobacilli and bifidobacteria are the major components of human microflora (microbiota). Representatives of both genuses can colonize the mucosa layer of the colon and affect homeostasis maintenance of the intestinal ecosystem. Their presence is associated with beneficial health effects such as suppression of harmful microorganisms, improvement of digestion, immune stimulating effect, reduction of lactose intolerance, reduction of serum cholesterol, prevention of diarrhea and others. Some strains of lactobacilli produce bioactive peptides that have antihypertensive effect as well as calcium- and magnesium-absorbing effect. In general, probiotic effects do not relate to the genus *Lactobacillus* and *Bifidobacterium,* but are strain specific. In order to prove beneficial effects an accurate identification of species and strains is carried out during studying. Many beneficial health effects are registered after the consumption of products containing probiotic strains. Below are listed the most important benefits:

- Favorable effect on the intestinal flora by stimulating beneficial bacteria and suppressing harmful microorganisms
  Several strains of lactic acid bacteria produce bacteriocins, which destroy harmful microorganisms such as *Clostridium tyrobutiricum, St. aureus, Listeria monocytogenes* and others.
- Production of short-chain fatty acids
  In a healthy human body are produced about 400 mM short-chain fatty acids per day, as the most important are: acetic acid, propionic acid and butyric acid. They favorably affect the pH in the intestine. It was found that butyrate reduces the risk of developing colon cancer by inhibiting the genotoxic effect of nitrosamines and hydrogen peroxide. Furthermore, butyric acid induces apoptosis in tumor cells of the colon. Fatty acid reduces the expression of proinflammatory cytokines TNF$\alpha$, IL-1$\beta$, IL-6.
- Stimulation of the immune response through immunomodulation and enhancement of inherited immunity
  Probiotic bacteria regulate the delicate balance between necessary and excessive immune response. Probiotic strains that lead to stimulation of the induction of IL-10 are suitable for people suffering from inflammatory disease of the colon. Immunomodulating effect is strain specific. Several strains of lactobacilli and bifidobacteria, some of which lead to reduction of TNF$\alpha$ and increase of IL-10 (McCarthy, 2003), are examined.
- Improvement of nutrients absorption in the macroorganism
  Lactobacilli and bifidobacteria have beta-galactosidase activity thus decreasing the lactose concentration. This is beneficial for people suffering from lactose intolerance. Production of B-complex vitamins by some bifidobacteria strains is significant and noted above. Lactobacilli contribute to the protein degradation through its proteolytic complex and thus facilitate the absorption of proteins by humans. Some bifidobacteria can assimilate inorganic nitrogen and subsequently can lower the concentration of ammonia, which can be carcinogenic at high levels.
- Antioxidant effect
  Increased accumulation of oxygen radicals in tissues can cause damage to cells. The formation of active oxygen may be associated with factors such as use of certain drugs, increased alcohol consumption, stress and other factors. Increased oxidation is associated with some digestive disorders as well as age, arteriosclerosis, cancerous diseases, etc. The body has several defenses against toxic oxygen, but it is important to consume antioxidants to improve the functioning of these systems. Some microorganisms possess antioxidant enzymes such as SOD (Superoxide dismutases), catalase enzymes, glutathione reductase enzymes and others.
- Reduction of serum cholesterol
  Cholesterol is synthesized by intestinal cells and released into the intestine. Another part of cholesterol is from the bile secretion and food. The microbiota affects cholesterol levels. Some microorganisms degrade bile salts, utilizing taurine and glycine. Thus, bile salt-circulation is obstructed and the liver mobilizes extra cholesterol for the synthesis of new bile salts, which leads to decreasing of serum cholesterol. Gilliland et al. (1985) studied the capacity of the strain *L. acidophilus* to assimilate cholesterol.
- Secretion of bioactive peptides with antihypertensive activity and peptides improving the absorption of calcium and magnesium.
  By breaking down milk proteins the lactic acid bacteria release low molecular weight peptides. It was found that some of them inhibit angiotensin-converting enzyme (ACE). ACE cleaves dipeptide from angiotensin I, converting it into angiotensin II (a vasoconstrictor that induces the aldosterone production), which leads to an increase in the concentration of sodium ions and the blood pressure. The best studied effect is the antihypertensive effect of oligopeptides Ile-Pro-Pro and Val-Pro-Pro that are released during proteolytic activity of certain strains of *L. helveticus.* They induce strong inhibition of ACE. Antihypertensive effect is confirmed in clinical trials.

Currently, there are several commercial probiotics that are used successfully, e.g. *L. rhamnosus* GG (Saxelin M. Lactobacillus GG - a human probiotic strain with thorough clinical documentation. Food Rev Int 1997, 13:293-313). Another industrial probiotic is *L. fermentum* 39, used for stabilization of the intestinal microflora during dysbacteriosis (1).

There is a bacterial strain *Lactobacillus fermentum* ME3, possessing SOD activity and activity against pathogens (Mikelsaar et al., 2007).

References 3 and 4, for example, disclose Lb. gasseri strains and other lactobacilli for probiotic and health related use. Reference 5 discloses the adherence to HT-29 Cells of several L. plantarum strains. A topical problem is the identification and selection of probiotic strains of lactic acid bacteria and using them for preparation of a polybacterial product that has a number of useful properties, such as high survival in the gastrointestinal tract, antioxidant effects, strong adhesion to the epithelial layer of the colon, cholesterol reduction capacity, immune modulating and anti-inflammatory effect, ACE inhibitory activity.

## DISCLOSURE OF THE INVENTION

The objective of this invention is to identify and select strains of probiotic lactic acid bacteria with beneficial health properties, which shall be used for the creation of a probiotic preparation that possesses a combination of functional effects.

The probiotic preparation, which is object of the present invention, is a polybacterial combination consisting of three probiotic strains of lactic acid bacteria: *Lactobacillus gasseri* 7/12, deposited under No. 8720 at the National Bank for Industrial Microorganisms and Cell Cultures (NBIMCC), *Lactobacillus plantarum* F12, deposited under No. 8722 at NBIMCC and *Lactobacillus helveticus* A1, deposited under No. 8721 at NBIMCC. The product has a number of health benefits: antioxidant effect; reduction of cholesterol concentration due to hydrolysis of bile salts and direct absorption of cholesterol; anti-inflammatory immune modulating effect by reduction of the level of the proinflammatory cytokine "interleukin-8"; capacity to release bioactive peptides inhibiting the enzyme responsible for the high blood pressure (ACE).

Objects of the invention are also new strains of *Lactobacillus gasseri* 7/12 NBIMCC No. 8720, *Lactobacillus plantarum.* F12 NBIMCC No. 8722 and *Lactobacillus helveticus* A1 NBIMCC No. 8721 and their beneficial properties.

The probiotic strain *Lactobacillus plantarum* F12 NBIMCC No. 8722 possesses both antioxidant activity and a significant adhesion to epithelial cells.

The probiotic strain *Lactobacillus gasseri* 7/12 NBIMCC No. 8720 possesses a set of the following useful properties: high survival in the gastrointestinal tract, strong adhesion to the epithelial layer of the colon, the capacity to reduce cholesterol and anti-inflammatory effects by reducing the level of proinflammatory cytokine "interleukin-8".

The probiotic strain *Lactobacillus helveticus* A1 NBIMCC No. 8721 possesses strong anti-ACE effect.

Object of the invention is the use of new probiotic strains and the probiotic polybacterial preparation in pharmaceutical and food industries in order to prevent socially significant diseases such as: hypertension, arteriosclerosis and hypercholesterolemia, weakened immunity and inflammation processes in the intestinal tract, disturbed balance in the intestinal microflora.

*Lactobacillus gasseri* 7/12 NBIMCC No. 8720 and *Lactobacillus plantarum* F12 NBIMCC No. 8722 are isolated from the intestinal tract of healthy volunteers. *Lactobacillus helveticus* A1 NBIMCC No. 8721 is isolated from homemade white cheese, which proves their GRAS status (generally recognized as safe). Species affiliation and strains identity of the three strains are established through a precise DNA-based methods (2) sequencing of the 16S ribosomal genes, ARDRA, pulse electrophoresis, AFLP.

Phenotypic identification of the three strains is performed using the API 50 CHL System (BioMerieux, France).

Strains are molecularly identified by means of AFLP and pulse electrophoresis. The two main molecular methods, which are used for identification of the three strains and the obtaining of their strain-specific DNA profiles, are described in the abovementioned article (Dimitrov Zh. Et al., 2008). In Figure 1 is represented pulse electrophoretic DNA profile of *Lactobacillus plantarum* F12, NBIMCC No. 8722, in Figure 2 - pulse electrophoretic DNA profile of *Lactobacillus gasseri* 7/12 NBIMCC No. 8720 and in Figure 3 - pulse electrophoretic DNA profile of *Lactobacillus helveticus* A1 NBIMCC No. 8721.

*Lactobacillus plantarum* F12 NBIMCC No. 8722 forms white S-colonies with sizes of 1-2 mm. Bacterial cells are short (0.9 to 1.2 X 3-8 um) with rounded corners. The major cultivation medium is MRS broth and agar (OXOID). The identification is performed according to the Bergey's Manual (2009) by using the Api web database (API 50CHL strips) and MicroLog M5.1.1 database (Biolog AN Microplate). ARDRA profile is typical for the strain *L. plantarum.* The profile of soluble cellular protein is identical to that of *L. plantarum* (ATCC). It grows in MRS broth after inoculation with 0.1%. The cultivation is conducted at 37 °C for about 18-24 hours. The broth medium and atmospheric air do not require any preliminary anaerobization during cultivation. The strain develops for 48h in an anaerobic environment on MRS agar.

*Lactobacillus gasseri* 7/12 NBIMCC No. 8720 forms white S-colonies with sizes of 1-2 mm. Bacterial cells are short (0, 6-0.8 X 3.0-5.0 um) with rounded corners. The major cultivation medium is MRS broth and agar (OXOID). The strain was identified according to the Bergey's Manual (2009) by using the Api web database (API 50CHL strips) and MicroLog M5.1.1 database (Biolog AN Microplate). ARDRA profile is typical for the strain *L. gasseri*. The profile of soluble cellular proteins is identical to that of strain *L. gasseri* 33323 (ATCC). *Lactobacillus gasseri* 7/12 grows on MRS broth after inoculation with 0.1%. The cultivation is conducted at 37 °C for about 18-24 hours. The broth medium and atmospheric air do not require any preliminary anaerobization during cultivation. The strain develops for 48h in an anaerobic environment on MRS agar.

*Lactobacillus helveticus* A1 NBIMCC No. 8721 forms white R-colonies with sizes of 1-2 mm. Bacterial cells have a length of 3,0-5,0 um. Basic culture media are MRS broth and agar (OXOID) and sterile skimmed milk for microbiological purposes, 10% dry matter (OXOID). The strain is identified according to the Bergey's Manual (2009) by using the Api web database (API 50CHL strips) and MicroLog M5.1.1 database (Biolog AN Microplate). ARDRA profile is typical for *L. helveticus*. The profile of soluble cellular proteins is identical to that of strain *L. helveticus* 15009 (ATCC). *Lactobacillus helveticus* A1 grows on MRS broth after inoculation with 0.1%. The cultivation is conducted at 37 °C for about 18-24 hours. The broth medium and atmospheric air do not require any preliminary anaerobization during cultivation. The strain develops for 48h in an anaerobic environment on MRS agar. The strain develops on sterile skimmed milk after inoculation with 0.1% and cultivated at 37 °C for 18-24 hours.

## Functional properties of strain *Lactobacillus plantarum* F12 NBIMCC No. 8722

### • Determination of antioxidant effect

The strain *L. plantarum* F12 has antioxidant properties and as such it is selected from among more than 400 lactobacilli. In order to make the selection, in terms of antioxidant properties, the strains are incubated in MRS broth for 24 hours and centrifuged at 3500 g for 10 min at 4 °C. After washing with saline solution the cells are re-suspend in 1.15% KC1 to $1 \times 10^9$ cells in 1 ml. Cell suspension is subjected to sonication (Branson B-12 Sonic power Company) for 5 min in an ice bath, followed by 10 min stay at -20 °C. The suspension is centrifuged at 10,000 g for 10 min at 4 °C. The supernatant is used as acellular extract. Three types of activities are determined:

### - Total antioxidant activity ORAC

The main method for determining total antioxidant activity is ORAC (Oxygen radical absorbance capacity). The result is presented in TROLOX equivalents of $10^9$ microbial cells. The *Cao et al* (1995) method is used. The principle is as follows: substrate OI-phycoerythrin (Sigma Chemical Co.) is subjected to oxidant attack by radical generator 2.2 v'Di-azobis (2-amidinopropane) dihydrochloride (AAPH; Waco Chemical USA). The standard solution TROLOX (6-hydroxy-2 ,5,7,8-tetramethylchroman-2-carboxylic acid, Trolox; Aldrich) is prepared by dissolving 5 mg of the substance in 200 ml of 0.2 M phosphate buffer (stock solution). The working solution is obtained by mixing 1 ml of the stock solution with 9 ml of the phosphate buffer.

In the presence of a sample with antioxidant properties the oxidation of phycoerythrin is reduced to some extent. The relative fluorescence is measured using waves with lengths of 535 nm (excitation) and 595 nm (emission), respectively, for a period of 60 minute. The following formula is used:

$$\text{ORAC value} = X \; K \; (S_{sample} - S_{blank}) / (S_{trolox} - S_{blank})$$

Where X is the sample volume in microliters, and K is the coefficient of dilution. S - the areas of fluorescent curve.

### - General antiradical activity

The substances that capture free radicals are determined for each of the strains based on the degree of reduction in the intensity of staining of the compound 1,1-diphenyl-2-picrilhydrazine (DPPH). The strength of the radical capturing activity is proportional to the degree of discoloration of the purple color of DPPH. The milk fermented with the corresponding strain is centrifuged and the supernatant is extracted with diethyl ether. The process is followed by evaporation of the ether layer and dissolving the residue in methanol. A volume of 0.1 ml of this solution is mixed with 1.4 ml of DPPH solution (40 $\mu$g/ml in methanol). Radical capturing activity is defined as a difference in absorbance at 517 nm between an empty sample and the sample [$A_{517}$ (empty) - $A_{517}$ (sample)].

- **SOD activity**

Test is performed pursuant to Chang & Hasan, 1997. The strains grow on broth (M17 for streptococci, MRS for lactobacilli) and after incubation the cultures are centrifuged, washed with phosphate buffer with 0.1 mM EDTA, pH 7.8, resuspended in the same buffer and then sonicated. Centrifuged at 17000g and dialyzed against the above phosphate buffer for 24 hours. Centrifuged again at 20000 g. The total protein concentration of acellular extract is determined pursuant to Lowry. Both enzymatic activities are defined using special test kits (SIGMA) according to the manufacturer's instructions. SOD is determined by the cytochrome C method in a conjugated enzyme system with xanthine oxidase. The principle is: xanthine oxidase oxidizes xanthine while cytochrome C is reduced. In the presence of a sample with a SOD activity the reduction of cytochrome C is inhibited to some extent, because the two protons bind to the superoxide radical from SOD instead to be transferred to cytochrome C.

Table 1 presents the results of determination of different antioxidant activities: total antioxidant activity by the ORAC test, total antiradical activity and SOD activity.

Table 1

| Activity | Intact cells | Acellular extract |
|---|---|---|
| Total antioxidant activity ORAC, trolox eq./$10^9$ cells | 1488 $\pm$ 72 n=5 | 1764 $\pm$ 110 n=5 |
| Total antiradical activity, $A_{517}$(empty) - $A_{517}$(sample) | 0.19 $\pm$ 0.02 n=5 | 0.32 $\pm$ 0.04 n=5 |
| Superoxide dismutase activity | | 38.4 $\pm$ 2.4 U/mg protein n=3 |

Significant total antioxidant activity is found both in intact cells of strain *L. plantarum F12,* as well as in the acellular extract. In terms of SOD activity acellular extract of this strain surpasses all tested lactobacilli from different bacterial species.

• **Determination of adhesion against epithelial cells**

Epithelial cell lines Caco-2 and HT29 are cultured as monolayers in DMEM medium (Dilbecco's modified Eagle's medium, Gibco, UK), filled with 10% fetal bovine serum. Incubation temperature is 37 °C in water saturated atmosphere containing 5% $CO_2$. At approximately 90% of a built monolayer the cells undergo passaging through incubation with 0.25% trypsin and 10 mM EDTA solution for 10 min at 37 °C. In order to determine adhesion the eukaryotic cells are seeded in 6-well cluster culture dish at a concentration of $2 \times 10^5$ cells/ml. The medium is changed every 2 days for a total of 10 days while the monolayer is built (3-4 days) and cell receptors are maturing. After adhesion the monolayers are washed twice with PBS buffer. Bacterial cultures at a concentration of 108 cells/ml in 2 ml DMEM without antibiotics are added to the monolayers. Incubation for 60 min at 37 °C in a saturated atmosphere containing 5% $CO_2$ is performed. Then the wells are washed five times with PBS and fixed with methanol for 3 minutes. Afterwards Gram staining and microscopic evaluation of adhesion is performed. The number of bacteria adhered to 20 epithelial cells is counted. Test includes reporting of 10 microscope fields.

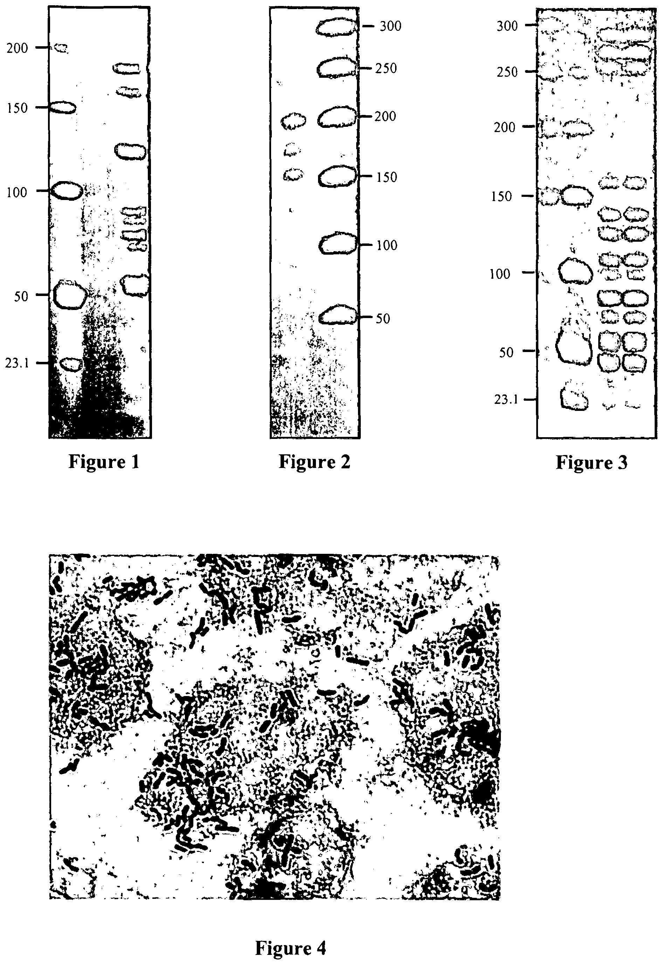

Figure 4 presents a picture of the adhesion of strain *L. plantarum* F12 to epithelial cells Caco-2. The results of adhesion of the strain, expressed as an average number of bacteria adhered to a eukaryotic cell, are presented in Table 2. The test is conducted three times in order to evaluate the adhesion on both cell lines Caco-2 and HT-29, respectively, and the results are averaged.

Table 2

| Strain | Average number of bacteria adhered to Caco-2 eukaryotic cell | Average number of bacteria adhered to HT-29 eukaryotic cell |
|---|---|---|
| *L. plantarum* F12 | 25 | 29 |

**Functional properties of strain *L. gasseri* 7/12 NBIMCC No. 8720**

**• Determination of adhesion against epithelial cells**

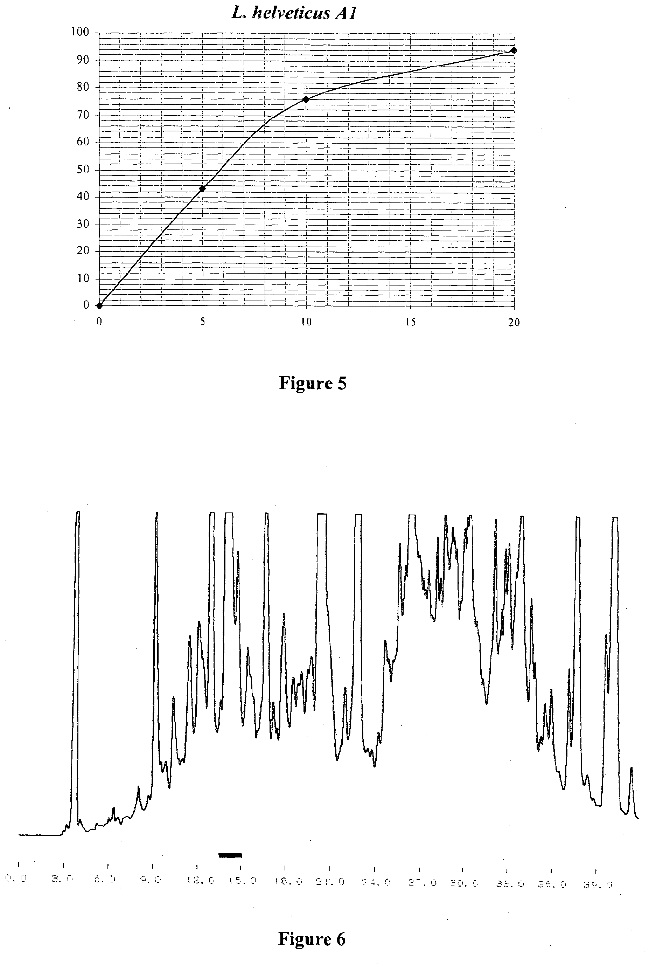

The method is shown above. Figure 5 shows a photograph of the adhesion of strain *L. gasseri* 7/12 to epithelial cells Caco-2. The results of adhesion of the strain, expressed as an average number of bacteria adhered to a eukaryotic cell, are presented in Table 3. The test is conducted three times in order to evaluate the adhesion on both cell lines Caco-2 and HT-29, respectively, and the results are averaged.

Table 3

| Strain | Average number of bacteria adhered to Caco-2 eukaryotic cell | Average number of bacteria adhered to HT-29 eukaryotic cell |
|---|---|---|
| *L. gasseri* 7/12 | 20 | 22 |

**• Determination of anti-inflammatory immunomodulating effect**

Boosting the induction of inflammatory cytokines is highly undesirable in people with inflammatory bowel disease (IBD). Synthesis of inflammatory cytokines is inhibited by the signal peptide Interleukin 10 (IL-10), which is produced by naive T-helper cells or macrophages. The human monocyte cell line U-937 proved to be suitable not only for evaluation of the induction of cytotoxic TNF-$\alpha$ and IL-1, but also in determining the induction of signal peptide IL-10.

The quantitative determination of the expression of the two signal peptide TNF-$\alpha$ and IL-10 of antigen-presenting cells is used for measuring the immunomodulating effect: differentiated macrophages of human monocyte cell line U-937 (ATCC). The maintenance of the cell line is done at 37 °C and 5% $CO_2$ on RPMI 1640 medium (ATCC 30-2001), containing 10% fetal bovine serum (ATCC 30-2020), 4.5 g/l glucose, 10mM HEPES, 1mM sodium pyruvate , 2mM L-glutamine, 1.5 g/l sodium bicarbonate, 100 U/ml penicillin, 100 $\mu$g/ml streptomycin. Maintained within the ranged of 2 x $10^5$ to 1 x $10^6$ cells per milliliter. Upon reaching a density of about 1 x $10^6$, cells are centrifuged and the medium is renovated. The cell content, which is reported by a cytometer, is 2 x $10^5$. In 48-well cluster culture dish 1 x $10^6$ cells per milliliter (5 x $10^5$ for the well) can be differentiated to macrophages with the aid of 5 $\mu$g/ml PMA (phorbol 12-myristate-13-acetate, SIGMA), added to the growth medium for 48 hours in a $CO_2$ incubator. Differentiated adhered macrophages are washed twice with PBS buffer (Dulbecco's phosphate-buffered saline, GibcoBRL) and incubated with fresh medium for 48 hours without PMA. After aspiration of the medium at each well with adhered macrophages are added 0.5 ml bacterial cells with a concentration of 1 x $10^6$ per ml, suspended in the growth medium of the macrophages. The cells are incubated for 24 hours in a $CO_2$ incubator.

- **Assessment of induction of the signal peptide TNF-**$\alpha$ of lactic acid bacteria is done by sandwich ELISA. At the end of incubation the supernatant is collected and centrifuged to eliminate bacterial and human cells. 200$\mu$l of the resulting supernatants are used for determination of TNF-$\alpha$ through a special kit of R&D Systems (Human TNF-$\alpha$/TNFSF1A Immunoassay, Cat. # DTA00C), following manufacturer's instructions. Briefly: immune dishes are covered with a monoclonal antibody against human TNF-$\alpha$, which holds TNF-$\alpha$ in a special manner. After washing, the wells are treated with a polyclonal antibody against TNF-$\alpha$, which is connected to the enzyme Horseradish Peroxidase. Afterwards are added the substrates, hydrogen peroxide and tetramethylbenzidine, and after an enzymatic reaction at room temperatures for 20 min at dark the reaction is topped with 2N sulfuric acid. The absorption is measured at 450 nm. For calculation of the TNF-$\alpha$ concentration is used standards and a standard curve is drawn.
- **Assessment of induction of the signal peptide IL-6** of lactic acid bacteria is done by sandwich ELISA. The principle of detection is analogous to that described above. During the assessment is used analytical ELISA kit and then quantitative evaluation is performed according to the manufacturer's instructions.
- **Assessment of induction of the signal peptide of IL-10** of lactic acid bacteria is done by sandwich ELISA. During the assessment is used analytical ELISA kit and then quantitative evaluation is performed according to the manufacturer's instructions.

The assessment of the five cytokines TGF-$\beta$, IL-8, IL-6, IL-10 and TNF$\alpha$ for each of the isolated strains is performed three times. Table 4 gives the average induction results for the five cytokines. The strain *L. gasseri* 7/12, which has a moderate adhesion, is the most successful anti-inflammatory strain among the other subjects. He has a good correlation between IL-10 and TNF-$\alpha$, a significant reduction of IL-8 and TGF-$\beta$, and has no significant induction of IL-6. Proinflammatory cytokine IL-8 is synthesized by epithelial cells. For the assessment are used cell lines Caco-2 and HT-29. The increased levels of IL-8 may lead to increased activity of neutrophil cells and hence to inflammatory problems in the colon. The discovery of strains that are able to reduce the level of IL-8 is essential for the establishment of bacterial preparations for the prevention of inflammatory diseases of the gastrointestinal tract. The strain *L. gasseri* 7/12 is valuable in this regard. The results of stimulation of epithelial cells with *L. gasseri* 7/12 are presented in Table 5.

Table 4

| Strain | IL-10 ng/ml | TNF-$\alpha$ ng/ml | TGF-$\beta$ ng/ml | IL-6 ng/ml |
|---|---|---|---|---|
| control (U-937) | 0.10 | 0.2 | 0.88 | 27.2 |
| *L. gasseri* 7/12 | 2.3 | 0.7 | 0.82 | 28.7 |

Table 5

| Strain | IL-8 ng/ml | TGF-$\beta$ ng/ml |
|---|---|---|
| control (HT-29) | 8.7 | 0.37 |
| *L. gasseri* 7/12 | 6.2 | 0.30 |

**• Determination of the capacity to reduce cholesterol levels**

**- Bile Salt Hydrolase (BSH) Activity**

Strains are grown under optimal conditions in broth media as given above, but in broths are added the two most important bile salts: sodium taurocholate (TCA) and sodium glycocholate (GCA). The concentration of salts is 1 mM. They are added after sterilization by microfiltration of the broth. The inoculum is 1% and incubation time is 24 hours. Uninoculated broth is used as a control. BSH activity is measured as deconjugated nanomoles TCA and GCA for 1 minute. The determination is high-performance liquid chromatography (HPLC). The column used is Ultrasphere ODS column, 80 angstrom, 15 cm, 3$\mu$m diameter of the filler grains. The configuration used is SHIMADZU UV detector at 210 nm. Free and conjugated bile acids are detected at a gradient reverse phase mode. Solvent A is 65% methanol in 0.03 M sodium acetate at pH 4.3, adjusted with phosphoric acid. Solvent B is methanol with a HPLC quality. The eluent program is an isocratic step for 8 minutes at 15% eluent B and then linear gradient to 85% for 17 minutes. The process is followed by an isocratic step of 5 minutes at 85%. The injected volume is 10 $\mu$l. BSH activity values are expressed in micromoles choline acid (CA) released from 1010 cells per minute and 24 hour incubation time.

**- Direct reduction of cholesterol**

The strains are inoculated at a concentration of 1% in the respective media for 24 hours. The broth media contain 0.3% Oxgal and 100 $\mu$g of water-soluble cholesterol per ml (cholesterol-PEG600, SIGMA). After incubation the cells are pelleted by centrifugation and 0.5 ml of the supernatant is treated with 4 ml of methanolic 2M KOH by heating for 20 minutes at 80 °C. It is extracted with 5 ml of hexane and 4 ml of the hexane layer is transferred into test tubes and evaporated with nitrogen at 60 °C. Dry residue is dissolve in 0.5 ml of isopropanol and the cholesterol content is determined with the analytical kit of Boeringer Manhaim. The degree of cholesterol reduction is determined on the basis of uninoculated control media.

Two main activities related to the reduction of cholesterol are examined - direct lowering of cholesterol levels and indirect cholesterol reduction of serum cholesterol as a consequence of deconjugating activity against bile salts (BSH). In the direct elimination of cholesterol, mainly due to the adsorption onto the microorganisms' cell wall components, the residual cholesterol is measured as described in the methodological part. Measuring the concentration of cholesterol is carried out using enzymatic test. Measurements are made three times and the results are averaged. Table 6 presents the results of direct anticholesterol activity expressed as percentage reduction of cholesterol.

Table 6.

| Strain | % cholesterol decrease |
|---|---|
| *L. gasseri* 7/12 | 36.2 |

BSH deconjugating activity indirectly affects on serum cholesterol levels. The strains with BSH activity hydrolyze bile salts and then deconjugated bile acids are transferred to the exit of the intestinal tract. Bile salts are recycled in the body, and after the reduction of their concentration in the liver, part of the cholesterol is switched to the production of bile acids and subsequently bile salts (bile acid conjugates with the amino acids taurine and glycine). In spite of all, the level of serum cholesterol is expected to drop. In Table 7 are given the results of BSH activity of *L. gasseri* 7/12 after threefold testing and averaging the results.

Table 7

| Strain | % deconjugation of bile salts |
|---|---|
| *L. gasseri* 7/12 | 60.1 |

Functional properties of strain *L. helveticus* A1 NBIMCC No. 8721

• **Determination of capacity for secretion of bioactive ACE-inhibiting peptides**

**- ACE-inhibiting activity**

Purified strains with inoculum of 0.1% are incubated for 18 hours in 9% skimmed milk at 37 °C. After adjusting the pH to 4.3 with 50% lactic acid the samples are centrifuged (5000 g, 10 min). Supernatant is dosed at reverse-phase cartridge and washed with water. The process is followed by elution with 60% acetonitrile in 0.1% trifluoroacetic acid and then evaporation and reconstitution of the resulting extract. The extract or its dilutions are analyzed for inhibition of ACE.

A total peptide extract is prepared. Enzymatic reaction is carried out by a conventional test, but using different dilutions of peptide extracts as their volume is 20 $\mu$l. The enzyme solution was 40 $\mu$l with an activity of 0.1 U/ml. The substrate hipuril-histidil-leucine is dissolved in borate buffer at pH 8.3 and has a volume of 190 $\mu$l. The final concentration of the substrate is 6 mM, and those of sodium chloride is 300 mM. The reaction is conducted at 37 °C for 30 minutes. The seizing is performed with 100 microliters of 4n HCl, and the extraction of the freed hippuric acid is done with 1 ml of ethyl acetate. After evaporation of the solvent, the residue is dissolved in 1 ml of water and tested spectrophotometrically at 228 nm.

The percentage of inhibition of ACE enzyme is reported by the formula:

$$\frac{B-A}{B-C}*100$$

where A is the absorbance under the upper reaction, C is the absorbance without enzyme, B is the absorbance without the involvement of peptide extracts.

Graphs are constructed and on the abscissa are shown microliters of the peptide extract - 20, 10, 5, and on the ordinate is shown inhibition as percentages. The strength of inhibitory effect is considered graphically as the volume of peptide extract that is causing 50% inhibition of the ACE enzyme. The smaller volume suggest of a greater ACE inhibiting capacity of the product. Table 8 provides important peptidases activities of the strain *L. helveticus* A1, which are largely based on the ability of this strain to release bioactive peptides. Table 9 provides ACE-inhibitory activity of *L. helveticus* A1 in % inhibition of ACE, rounded to the nearest integer.

Table 8

| STRAIN | Aminopeptidase N (PepN) | X-prolyl-aminopeptidase (PepX) | Glu-aminopeptidase (PepA) | Pro-imino-peptidase (PIP) | Oligo-peptidase | peptidase | Protease |
|---|---|---|---|---|---|---|---|
| *L. helveticus* A1 | 690 | 405 | 3 | 14 | 7 | 2 | 10 |

Table 9

| Strain | % ACE-inhibiting |
|---|---|
| *L. helveticus* A1 | 88% |

For *L. helveticus* A1 is drawn a graph (Figure 5), which defined the inhibitory effect of dilutions 1/2 and 1/4 of the supernatants analyzed. The main objective is to detect the strength of anti-ACE effect on dilution of samples. On the abscissa are shown microliters of the supernatant used in the trial, and the ordinate shows the percentage of inhibition of ACE.

The results show that the strain *L. helveticus* A1 has a strong anti-ACE effect.

**- Determination of the sequence of ACE-inhibiting peptides from the strain *L. helveticus* A1**

Preparation of concentrated peptide extracts

The supernatants of each strain are subjected to centrifugal ultrafiltration through special ultrafiltration cartridges with a molecular mass cutoff of 5000 Da. Low molecular weight hydrophobic and medium hydrophobic peptides, among which is usually looking for anti-ACE representatives, are concentrate on reverse-phase cartridges. In particular, after ultrafiltration of the samples is added trifluoroacetic acid to 0.1% and the mixture is passed through the cartridges. Peptides are retained. After washing with 0.1% TFA follows elution of low molecular weight peptides with 60% acetonitrile in 0.1% TFA. After evaporation the peptides are re-suspend in 10% acetonitrile in 0.1% TFA and dosed on a preparative reverse-phase HPLC column Nucleosil C18. The high-performance liquid chromatography device SHIMADZU is equipped with two pumps, HPLC column module with the ability to maintain a certain temperature, absorption detector and a fraction collector.

In order to verify the activity, all concentrates are measured for anti-ACE activity.

Coarse fractionation of peptide concentrates after chromatographic separation.

Samples injected into the HPLC column, as indicated above, are subjected to reverse-phase gradient fractionation. The acetonitrile gradient is from 0% to 80% in 0.1% TFA. Peptides are detected at a wavelength of 214 nm. By means of a fraction collector are captured fractions of 1 ml. After coarse fractionation these fractions are evaporated in a vacuum centrifuge and re-suspend in 0.1% TFA. The anti-ACE activity of the fractions is tested in order to be selected those fractions that have such activity. The chromatogram that shows the coarse fractionation of peptide concentration of strain *L. helveticus* A1 is shown in Figure 6.

Refractionation of selected fractions for collecting purified peptides after high performance reversed phase chromatography.

Methodical fine fractionation is performed on the same principle as the coarse fractionation - i.e. reversed phase, but the slope of the gradient is gentler. Gradient conditions for fractions of the strain *L. helveticus* A1 are 25-35% acetonitrile. The column used is semi-preparative Nucleodur Sphinx.

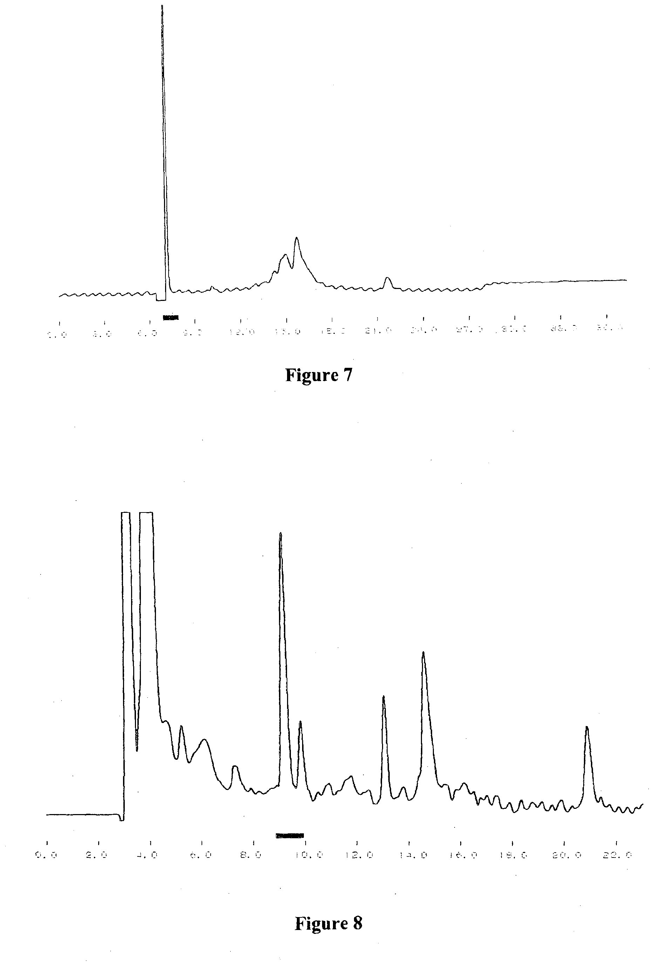

In Figure 7 is shown a chromatogram of the fine reverse-phase refractionation samples of the pre-selected coarse peptide fractions for the strain *L. helveticus* A1. The active anti-ACE fraction after conducting the enzyme test for inhibition of ACE is marked with a bold line. The final step for purification of the selected peptide fractions is purification through ion exchange HPLC column. The column is highly acidic cation-exchange column with benzensulfonate ion-active groups in a lithium form. Elution is achieved by a pH gradient in a citrate buffer at pH 3.0 to 9.0.

In Figure 7 is shown a chromatogram of the ion-exchange treatment for pre-selected active anti-ACE fraction of the strain *L.helveticus* A1. With a bold line is marked the single peptide with anti-ACE activity.

Peptide sequencing

The principle of sequencing is the classical scheme of manual sequencing with phenylisothiocyanate: starting with phenyl thioisocyanate: initial derivatization of amino-terminus of the peptide; cutting off of derivative N-terminal amino acid (CA) using 100% trifluoroacetic acid (TFA); separation by extraction of derivative N-terminal amino acid from the residual peptide; conversion of N-terminal amino acid to phenylthiohydantoin derivative by treatment with 40% (TFA); HPLC analysis of the PTH-amino acid; next cycle for the new N-terminal amino acid. In case of small hydrophobic peptides, the step of separation by extraction of derivative N-terminal amino acid from the residual peptide is impossible. This can be done by preliminary fixing of the C-terminal of the peptide to arylamine-PVDF membrane. Thus, peptide can be sequenced to the last C-terminal amino acids from the N-terminus. Fixing of the peptide from the COOH-terminus is possible after activation of the COOH-terminus by treatment with EDC (ethyl-dimethylaminopropyl carbodiimide) and subsequent reaction of activated carboxyl groups with the arylamine molecule of the membrane. Table 10 presents the sequence of anti-ACE peptides released by the strain *L. helveticus* A1

Table 10

| Strain | Anti-ACE activity % | Peptide sequence |
|---|---|---|
| *L. helveticus* A1 | 78.0 | Ala-Leu-Pro-Met |

## DESCRIPTION OF THE DRAWINGS

**Figure 1:** Pulse-electrophoretic strain-specific DNA profile of *L. plantarum* F12 after hydrolysis with the enzyme *XhoI.* and impulses 1 s - 12 s for 24 hours at 5 volts/centimeter. Molecular marker - λ-DNA HindIII (0.1-200 kbp).

**Figure 2:** Pulse-electrophoretic strain-specific DNA profile of *L. gasseri 7/12* after hydrolysis with the enzyme *ApaI.* and impulses 2 s - 28 s for 24 hours at 5 volts/centimeter. Molecular marker - λ-Ladder DNA (50-1000 kbp).

**Figure 3:** Pulse-electrophoretic strain-specific DNA profile of *L.helveticus* A1 after hydrolysis with the enzyme *SmaI.* and impulses 5 s - 30 s for 26 hours at 5.2 volts/centimeter. Molecular marker - λ-Ladder DNA (50-1000 kbp) and λ-DNA HindIII (0.1-200 kbp).

**Figure 4:** Adhesion of *L. plantarum* F12 to epithelial cells Caco-2.

**Figure 5:** Diagram of ACE-inhibiting effect of *L. helveticus* A1. On the abscissa - μl peptide extract, on the ordinate - % ACE-inhibition

**Figure 6:** Coarse fractionation of peptide concentrate of *L. helveticus* A1

**Figure 7:** Fine reverse phase purification of peptide fraction of preliminary fractionation with the highest anti-ACE activity for the strain *L. helveticus* A1.

**Figure 8:** Ion-exchange purification of the active fraction of the preceding purification cycle for the strain *L. helveticus* A1.

## MODE FOR CARRYING OUT THE INVENTION

Polybacterial preparation contains lyophilized strains of *Lactobacillus gasseri* 7/12 NBIMCC No. 8720, *Lactobacillus plantarum* F12 NBIMCC No. 8722 and *Lactobacillus helveticus* A1 NBIMCC No. 8721. The three strains are grown separately in laboratory fermentation chambers, 5 liters each, for biomass accumulation at milk as a medium, which is hydrolyzed with alkaline protease, supplemented with 0.2% yeast autolysate for nutritive purposes (Springer Biotec), as the amount of the inoculum is 1% and pH is maintained at 5.7 with sodium hydroxide. Incubation continues till the beginning of the stationary phase. With the three cultures containing enriched biomass of about $10^9$ for *Lactobacillus plantarum* F12, and *Lactobacillus helveticus* A1, and $2 \times 10^8$ for *Lactobacillus gasseri* 7/12, an industrial fermenting agent is inoculated in a milk medium that is hydrolyzed with alkaline protease by the addition of 0.2% yeast autolysate for nutritive purposes (Springer Biotec), as the inoculum quantity is 2%. The pH is maintained at 5.7 with sodium hydroxide. The resulting biomass mixture of the three probiotic strains is dosed in an industrial lyophilizer. Sublimation drying is conducted at -28 to -29 °C. The final heating is up to 30 °C. Residual moisture is 4%. The mixture is packed in an inert (nitrogen) atmosphere. Special measures to reduce the mortality of the strains during the technological cycle are undertaken. The parameters of the fermentation, lyophilization and packaging are optimized. Survival and activity of the strains is controlled by means of modern molecular biology techniques (real-time PCR). The presence and strength of probiotic effects are controlled with high-precision techniques - High-performance liquid chromatography (hydrolysis of bile salts, analysis of bioactive peptides), Gas chromatography (determination of cholesterol-reducing activity), Real-time PCR (determining the vitality of the strains and their number), Spectral equipment (determination of antioxidant activities, detection of immunomodulating activities).

The ready-for-use lyophilized polybacterial preparation contains live bacteria (number of live cells per 1 g) of the three probiotic strains of at least: $10^9$ of *Lactobacillus gasseri* 7/12, $2 \times 10^9$ of *Lactobacillus helveticus* A1, $9 \times 10^9$ of *Lactobacillus plantarum* F12.

In Table 11 are given the results of determination of specific antioxidant activities such as: total antioxidant activity, total antiradical activity, SOD activity of the finished product.

Table 11

| Activity | Intact cells | Acellular extract |
|---|---|---|
| Total antioxidant activity ORAC, trolox eq./$10^9$ cells | $1105 \pm 68$ n=3 | $1320 \pm 95$ n=3 |
| Total antiradical activity, $A_{517}$(empty) - $A_{517}$(sample) | $0.12 \pm 0.02$ n=3 | $0.22 \pm 0.03$ n=3 |
| Superoxide dismutase activity | | $21.2 \pm 2.0$ U/mg protein n=3 |

In Table 12 and Table 13 are given the cytokine levels after induction of the human cell lines U-937 and HT-29 by $10^8$ /ml bacterial cells of the preparation.

Table 12

| Product | IL-10 ng/ml | TNF-$\alpha$ ng/ml | TGF-$\beta$ ng/ml | IL-6 ng/ml |
|---|---|---|---|---|
| Control (U-937) | 0.11 | 0.22 | 0.90 | 25.1 |
| Polybacterial preparation | 1.1 | 0.24 | 0.87 | 18.1 |

Table 13

| Product | IL-8 ng/ml | TGF-$\beta$ ng/ml |
|---|---|---|
| Control (HT-29) | 8.9 | 0.40 |
| Polybacterial preparation | 7.7 | 0.35 |

In Table 14 are given anti-cholesterol activities of the polybacterial preparation regarding the capacity for hydrolysis of bile salts and the decrease of cholesterol in percentages.

Table 14

| Product | % cholesterol decrease | % deconjugation of bile salts |
|---|---|---|
| Polybacterial preparation | 21.0 | 42.4 |

In Table 15 is given ACE-inhibiting activity of the preparation.

Table 15

| Product | % ACE-inhibition |
|---|---|
| Polybacterial preparation | 65% |

**INDUSTRIAL APPLICABILITY**

Polybacterial preparation is lyophilized dietary supplement with a unique combination of probiotic properties suitable for the prevention of socially significant diseases such as hypertension, arteriosclerosis and hypercholesterolemia, weakened immunity and inflammation processes in the intestinal tract, disturbed balance in the intestinal microflora. The production of this product requires the sharing of advanced industrial equipment and high-tech research equipment. The principle of production of the product is described in the example.

References:

1. PCT/SU89/00264, C12N1/20, A61K35/74, University of Tartu, 1991
2. Dimitrov Zh. et al., 2008. Comparative evaluation of three molecular typing methods in their applicability to differentiate Lactobacillus strains with human origin. World Journal of Microbiology and Biotechnology 24:1305-1312.

3. US 2008/019954 "Lactic acid bacteria strains useful against urogenital pathogens and compositions containing same".
4. USMAN ET AL: "Bile Tolerance, Taurocholate Deconjugation, and Binding of Cholesterol by Lactobacillus gasseri Strains ",JOURNAL OF DAIRY SCIENCE, vol. 82, no. 2, 1 February 1999 (1999-02-01), pages 243-248.
5. US 6159465 A "Epithelial Adhesive Lactobacilli".

**Claims**

1. Polybacterial preparation, **characterized by** the fact that it includes a combination of strains *Lactobacillus gasseri* 7/12 NBIMCC No. 8720 and *Lactobacillus plantarum* F12 NBIMCC No. 8722 and *Lactobacillus helveticus* A1 NBIMCC No. 8721, containing living cells measured in cfu/g as follows for: *Lactobacillus gasseri* 7/12 at least $10^9$, *Lactobacillus helveticus* A1 at least $2\times10^9$ and *Lactobacillus plantarum* F12 at least $9\times10^9$.

2. Polybacterial preparation according to claim 1, **characterized by** the fact that *Lactobacillus* gasseri 7/12 NBIMCC No. 8720 having an adhesive capacity of about 22 to HT-29 cell lines, *Lactobacillus plantarum* F12 NBIMCC No. 8722 having an adhesive capacity of about 29 to HT-29 cell lines and *Lactobacillus helveticus* A1 NBIMCC No. 8721 releasing peptide sequence Ala-Leu-Pro-Met.

3. Polybacterial preparation according to claim 1, **characterized by** the fact that it possesses antioxidant, anti-inflammatory immunomodulating effect, hypocholesterolemic effect and antihypertensive effect.

4. Polybacterial preparation according to claim 1 **characterized by** its form - lyophilisate or a liquid culture.

5. Bacterial preparation, **characterized by** the fact that it contains strain *Lactobacillus gasseri* 7/12 NBIMCC No. 8720.

6. Bacterial preparation of strain *Lactobacillus gasseri* 7/12 NBIMCC No.8720 according to claim 5, possessing hypocholesterolemic activity and anti-inflammatory immunomodulating activity and adhesive capacity of about 22 to HT-29 cell lines.

7. Bacterial preparation according to claim 5 **characterized by** its form - lyophilisate or a liquid culture.

8. Method for obtaining polybacterial preparation according to claim 1, comprising growing of *Lactobacillus gasseri* 7/12 NBIMCC No. 8720, *Lactobacillus plantarum* F12 NBIMCC No. 8722 and *Lactobacillus helveticus* A1 NBIMCC No. 8721, separately in a medium consisting of milk, hydrolyzed with alkaline protease, and a supplement of 0.2% yeast autolysate for nutritive purposes, as the amount of the inoculum is 1% and pH is maintained at 5.7 with sodium hydroxide and subsequent cultivation of the combination of the three monocultures contained enriched biomass of *Lactobacillus plantarum* F12, *Lactobacillus helveticus* A1 and *Lactobacillus gasseri* 7/12 in a medium consisting of milk, hydrolyzed with alkaline protease, and a supplement of 0.2% yeast autolysate for nutritive purposes, as the amount of the inoculum is 2% and pH is maintained at 5.7 with sodium hydroxide

9. Use of a polybacterial preparation of claim 1 as probiotic.

10. Use according to claim 9, where the probiotic is a food additive and / or included in any form in foods for humans and animals

11. Use according to claim 9, where the probiotic is for obtaining starter cultures for food production.

12. Use according to claim 9, where the probiotic is in the composition of functional products and pharmaceutical preparations intended to influence the health of humans and animals.

13. Use of a bacterial preparation of claim 5 as probiotic.

14. Use according to claim 13, where the probiotic is a food additive and / or included in any form in foods for humans and animals

15. Use according to claim 13, where the probiotic is for obtaining starter cultures for food production.

16. Use according to claim 13, where the probiotic is in the composition of functional products and pharmaceutical preparations intended to influence the health of humans and animals.

**Patentansprüche**

1. Polybakterienpräparat, **dadurch gekennzeichnet, dass** es eine Kombination aus den Stämmen *Lactobacillus gasseri* 7/12, NBIMCC No. 8720, *Lactobacillus plantarum* F12, NBIMCC No. 8722, *Lactobacillus helveticus* A1, NBIMCC No. 8721 umfasst, das folgende lebende Zellen in cfu/g enthält für: *Lactobacillus gasseri* 7/12 mindestens $10^9$, *Lactobacillus helveticus* A1 mindestens $2 \times 10^9$ und *Lactobacillus plantarum* F12 mindestens $9 \times 10^9$.

2. Polybakterienpräparat gemäß Anspruch 1, **dadurch gekennzeichnet, dass** *Lactobacillus* gasseri 7/12 NBIMCC No. 8720 eine Adhäsionskapazität von etwa 22 an der Zelllinie HT-29 hat, *Lactobacillus plantarum* F12 NBIMCC No. 8722 eine Adhäsionskapazität von etwa 29 an der Zelllinie HT-29 hat und *Lactobacillus helveticus* A1 NBIMCC No. 8721 die Peptidsequenz Ala-Leu-Pro-Met freisetzt.

3. Polybakterienpräparat gemäß Anspruch 1, **dadurch gekennzeichnet, dass** es einen antioxidativen, entzündungshemmenden immunmodulierenden Effekt, einen hypocholesterinämischen Effekt und einen antihypertensiven Effekt besitzt.

4. Polybakterienpräparat gemäß Anspruch 1, **gekennzeichnet durch** seine Form - Lyophilisat oder flüssige Kultur.

5. Bakterienpräparat, **dadurch gekennzeichnet, dass** es den Stamm *Lactobacillus gasseri* 7/12, NBIMCC No. 8720 enthält.

6. Bakterienpräparat des Stammes *Lactobacillus gasseri* 7/12, NBIMCC No. 8720 gemäß Anspruch 5, das eine hypocholesterinämische Aktivität und eine entzündungshemmende immunmodulierende Aktivität und eine Adhäsionskapazität von etwa 22 an der Zelllinie HT-29 besitzt.

7. Bakterienpräparat gemäß Anspruch 5, **gekennzeichnet durch** seine Form - Lyophilisat oder flüssige Kultur.

8. Verfahren zur Herstellung des Polybakterienpräparates gemäß Anspruch 1, umfassend die Kultivierung von *Lactobacillus gasseri* 7/12 NBIMCC No. 8720, *Lactobacillus plantarum* F12 NBIMCC No. 8722 und *Lactobacillus helveticus* A1 NBIMCC No. 8721 separat in einem Medium, das aus mit alkalischer Protease hydrolysierter Milch mit einem Zusatz von 0.2% Hefeautolysat für Nahrungszwecke besteht, wobei die Inokulummenge 1% beträgt, und der pH-Wert von 5.7 mit Natriumhydroxid und einer nachfolgenden Kultivierung der Kombination aus den drei Monokulturen aufrechterhalten wird, die angereicherte Biomasse aus *Lactobacillus plantarum* F12, *Lactobacillus helveticus* A1 und *Lactobacillus gasseri* 7/12 in einem Medium aus mit alkalischer Protease hydrolysierter Milch mit einem Zusatz von 0.2% Hefeautolysat für Nahrungszwecke, wobei die Inokulummenge 1% beträgt, und der pH-Wert von 5.7 mit Natriumhydroxid aufrechterhalten wird.

9. Verwendung des Polybakterienpräparates gemäß Anspruch 1 als Probiotikum.

10. Verwendung gemäß Anspruch 9, wo das Probiotikum ein Nahrungsergänzungsmittel ist und/oder in jeglicher Form in den Nahrungsmitteln für Menschen und Tiere enthalten ist.

11. Verwendung gemäß Anspruch 9, wo das Probiotikum zur Herstellung von Starterkulturen für die Produktion von Nahrungsmitteln verwendet wird.

12. Verwendung gemäß Anspruch 9, wo das Probiotikum in der Zusammensetzung von funktionellen Produkten und pharmazeutischen Präparaten zur Beeinflussung des Gesundheitszustandes von Menschen und Tieren enthalten ist.

13. Verwendung des Bakterienpräparates gemäß Anspruch 5 als Probiotikum.

14. Verwendung gemäß Anspruch 13, wo das Probiotikum ein Nahrungsergänzungsmittel ist und/oder in jeglicher Form in Nahrungsmitteln für Menschen und Tiere enthalten ist.

**15.** Verwendung gemäß Anspruch 13, wo das Probiotikum zur Herstellung von Starterkulturen für die Produktion von Nahrungsmitteln verwendet wird.

**16.** Verwendung gemäß Anspruch 9, wo das Probiotikum in der Zusammensetzung von funktionellen Produkten und pharmazeutischen Präparaten zur Beeinflussung des Gesundheitszustandes von Menschen und Tieren enthalten ist.


**Revendications**

**1.** Préparation polybactérienne, **se caractérisant par le fait qu'**elle inclut une combinaison des souches *Lactobacillus gasseri* 7/12, BNMICC № 8720, *Lactobacillus plantarum* F12, BNMICC № 8722, *Lactobacillus helveticus* A1, BNMICC № 8721, contenant des cellules vivantes dans cfu/g, comme il suit pour: *Lactobacillus gasseri* 7/12 au moins $10^9$, *Lactobacillus helveticus* A1 au moins $2 \times 10^9$ и *Lactobacillus plantarum* F12 au moins $9 \times 10^9$.

**2.** Préparation polybactérienne, conformément à la prétention 1, **se caractérisant par le fait que** *Lactobacillus* gasseri 7/12 BNMICC No. 8720 a une capacité d'adhésion d'environ 22 sur la ligne cellulaire HT-29, *Lactobacillus plantarum* F12 BNMICC No. 8722 a une capacité d'adhésion d'environ 29 sur la ligne cellulaire HT-29 et *Lactobacillus helveticus* A1 BNMICC No. 8721 libère la séquence péptidique Ala-Leu-Pro-Met.

**3.** Préparation polybactérienne, conformément à la réclamation 1, **se caractérisant par le fait qu'**elle possède un effet antoxydant, immunomodulateur, anti-inflammatoire, un effet hypocolestérolémiant et un effet antihypertenseur.

**4.** Préparation polybactérienne, conformément à la réclamation 1, **se caractérisant par** sa forme - lyophilisat ou culture liquide

**5.** Préparation bactérienne, **se caractérisant par le fait qu'**elle contient la souche *Lactobacillus gasseri* 7/12, BNMICC № 8720

**6.** Préparation bactérienne de la souche *Lactobacillus gasseri* 7/12, BNMICC № 8720 conformément à la réclamation 5, possédant une activité hypocholestérolémiante et une activité immunomodulatrice anti-inflammatoire, ainsi qu'une capacité d'adhésion d'environ 22 sur la ligne cellulaire HT-29.

**7.** Préparation bactérienne, conformément à la réclamation 5, **se caractérisant par** sa forme - lyophilisat ou culture liquide.

**8.** Méthode pour l'obtention d'une préparation polybactérienne conformément à la réclamation 1, qui comprend la cultivation de *Lactobacillus gasseri 7/12* BNMICC № 8720, *Lactobacillus plantarum* F12 BNMICC № 8722 et *Lactobacillus helveticus* A1 BNMICC № 8721 séparément dans un milieu composé du lait hydrolysé par de la protéase alcaline avec addition de 0.2% d'autolysat de levure à des fins nutritionnels, la quantité de semence étant de 1%, et le pH est maintenu à 5.7 avec de l'hydroxyde de sodium et la cultivation ultérieure des trois monocultures contenant la riche biomasse de *Lactobacillus plantarum* F12, *Lactobacillus helveticus* A1 et *Lactobacillus gasseri* 7/12 dans un milieu composé de lait hydrolysé par de la protéase alcaline avec addition de 0.2% d'autolysat de levure à des fins nutritionnels, la quantité de semence étant de 2%, et le pH est mantenu à 5.7 avec de l'hydroxyde de sodium.

**9.** Utilisation de préparation polybactérienne, conformément à la réclamation 1, comme probiotique

**10.** Utilisation, conformément à la réclamation 9, où le probiotique est un complément alimentaire et/ou inclus sous quelque forme que soit dans des aliments pour des humains et des animaux.

**11.** Utilisation, conformément à la réclamation 9, où le probiotique est pour l'obtention de cultures de départ pour la production alimentaire.

**12.** Utilisation, conformément à la réclamation 9, où le probiotique est dans la composition de produits fonctionnels et dans des préparatons pharmaceutiques destinés à avoir une influence sur la santé des humains et des animaux.

**13.** Utilisation de préparation bactérienne, conformément à la réclamation 5, comme probiotique.

**14.** Utilisation, conformément à la réclamation 13, où le probiotique est un complément alimentaire et/ou inclus sous quelque forme que soit dans des aliments pour des humains et des animaux

**15.** Utilisation, conformément à la réclamation 13, où le probiotique est pour l'obtention de cultures de départ pour la production alimentaire.

**16.** Utilisation, conformément à la réclamation 9, où le probiotique est dans la composition de produits fonctionnels et dans des préparatons pharmaceutiques destinés à avoir une influence sur la santé des humains et des animaux.

**Figure 1**

**Figure 2**

**Figure 3**

**Figure 4**

*L. helveticus A1*

**Figure 5**

**Figure 6**

**Figure 7**

**Figure 8**

20

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- SU 8900264 W
- US 2008019954 A
- US 6159465 A

**Non-patent literature cited in the description**

- **SAXELIN M.** Lactobacillus GG - a human probiotic strain with thorough clinical documentation. *Food Rev Int,* 1997, vol. 13, 293-313
- **DIMITROV ZH. et al.** Comparative evaluation of three molecular typing methods in their applicability to differentiate Lactobacillus strains with human origin. *World Journal of Microbiology and Biotechnology,* 2008, vol. 24, 1305-1312
- **USMAN et al.** Bile Tolerance, Taurocholate Deconjugation, and Binding of Cholesterol by Lactobacillus gasseri Strains. *JOURNAL OF DAIRY SCIENCE,* 01 February 1999, vol. 82 (2), 243-248